# EUROPEAN PATENT APPLICATION

(11) **EP 1 340 499 A1**
(43) Date of publication of application: **03.09.2003**
(21) Application number: 01999372.4
(22) Date of filing: 16.11.2001
(51) Int. Cl.: A61K 31/495, A61K 31/4468, A61P 25/28, A61P 43/00, C07D 295/32

(54) **NOOTROPIC EFFECT ENHANCER**

(30) Priority: 07.12.2000 JP 2000373260
(71) Applicant: FUJISAWA PHARMACEUTICAL CO., LTD., Osaka-shi Osaka 541-8514 (JP)
(72) Inventor: MATSUOKA, Nobuya, c/o Fujisawa Pharm. Co., Ltd, Osaka-shi, Osaka 541-8514 (JP); TOKITA, Kenichi, c/o Fujisawa Pharm. Co., Ltd, Osaka-shi, Osaka 541-8514 (JP)
(74) Representative: Gille Hrabal Struck Neidlein Prop Roos
(86) International application number: JP0110068
(87) International publication number: WO02045715

(57) **Abstract**

The invention is to provide an antidementic and/or antiamnesic potency enhancer that comprises an aminopiperazine or aminopiperidine derivative of a formula: 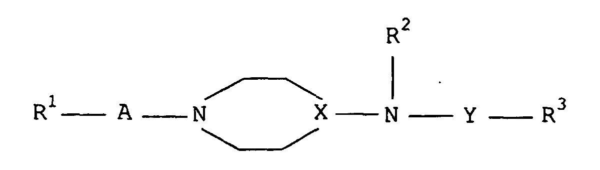 (wherein R¹ is a lower alkyl, etc.; R² is hydrogen, etc.; R³ is an aryl, etc.; A is -CO-, etc.; X is N or CH; Y is -CO-, etc.), for attaining higher antidementic and/or antiamnesic potency.

## Description

### TECHNICAL FIELD

The present invention relates to an antidementic and/or antiamnesic potency enhancer that comprises an aminopiperazine or aminopiperidine derivative.

More precisely, the invention relates to such an antidementic and/or antiamnesic potency enhancer that comprises an aminopiperazine or aminopiperidine derivative, which significantly enhances the antidementic and/or antiamnesic potency of other antidementic drugs such as donepedil hydrochloride (hereinafter referred to as "E2020") of which the function and the mechanism differ from those of the antidementic and/or antiamnesic potency of the aminopiperazine or aminopiperidine derivative.

### BACKGROUND ART

The aminopiperazine or aminopiperidine derivatives for use in the invention are known substances, and it is known that they have strong antidementic and/or antiamnesic potency (International Publication Nos. WO91/01979, WO98/25914 and WO00/42011). Further, E2020 for use in the invention, which is represented by the following structural formula, is also known [THE MERCK INDEX (12 edition), No. 3533], and it is available on the market in Japan and other countries as an antidementic having an acetylcholinesterase-inhibiting activity.

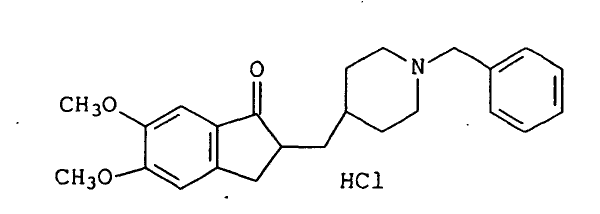

### Donepedil Hydrochloride (E2020)

We are now being in a superaging society and various diseases are increasing around us. Above all, senile dementia is increasing year by year. At present, some antidementic and/or antiamnesic drugs such as E2020 are available on the market, but more improved drugs with enhanced potency or those with reduced side effect are desired.

### DISCLOSURE OF THE INVENTION

We, the present inventors have specifically noted the function and the mechanism of aminopiperazine or aminopiperidine derivatives that have antidementic and/or antiamnesic potency, or that is, the ability of those derivatives to promote somatostatin release, and have found that the aminopiperazine or aminopiperidine derivatives (I) have the ability to significantly enhance the antidementic and/or antiamnesic potency of other antidementics that differ from those derivatives in their function and mechanism. On the basis of this finding, we have completed the present invention.

The aminopiperazine or aminopiperidine derivatives for use in the invention that have the ability to enhance antidementic and/or antiamnesic potency are represented by the following chemical formula (I): 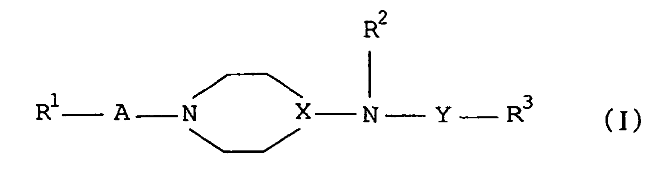 wherein R¹ represents a lower alkyl or an aryl; R² represents hydrogen or a lower alkyl; R³ represents an aryl that may be substituted with a halogen; A represents -CO- or -SO₂-; X represents N or CH; Y represents -CO- or -SO₂-.

Specific examples of the above-mentioned definitions and. preferred embodiments thereof are described below.

Unless otherwise specifically indicated, the term "lower" as referred to herein means from 1 to 6 carbon atoms.

Preferably, the "lower alkyl" is a linear or branched C₁-C₆ alkyl, including methyl, ethyl, propyl, isopropyl, butyl, isobutyl, tert-butyl, pentyl, ethylpropyl, hexyl, etc. Methyl is more preferred.

Preferred examples of the "aryl" are phenyl, naphthyl, lower alkyl-substituted phenyl [e.g., tolyl, xylyl, mesityl, cumenyl, di(tert-butyl)phenyl, etc.], etc. Phenyl is more preferred.

Preferred examples of the "halogen" are fluorine, chlorine, bromine and iodine. Fluorine is more preferred.

The aminopiperazine or aminopiperidine derivatives of formula (I) for use in the invention may include one or more stereoisomers such as optical isomers based on the asymmetric carbon atom therein, and all these isomers and their mixtures are within the scope of the invention.

Further, solvate compounds [for example, clathrate compounds (e.g., hydrates, etc.)] of the derivatives of formula (I) or their salts are also within the scope of the invention.

Salts of formula (I) are biologically-acceptable, generally nontoxic salts, and include, for example, acid-addition salts such as inorganic acid-addition salts [e.g., hydrochlorides, hydrobromides, sulfates, phosphates, etc.] and organic acid-addition salts [e.g., formates, acetates, trifluoroacetates, fumarates, maleates, tartrates, methanesulfonates, benzenesulfonates, toluenesulfonates, etc.], etc.

Of the aminopiperazine or aminopiperidine derivatives of formula (I) for use in the invention, the most preferred is a compound having the following chemical structure in which R¹ is methyl, R² is hydrogen, R³ is fluorine-substituted phenyl, A is -CO-, X is N, and Y is -CO-. 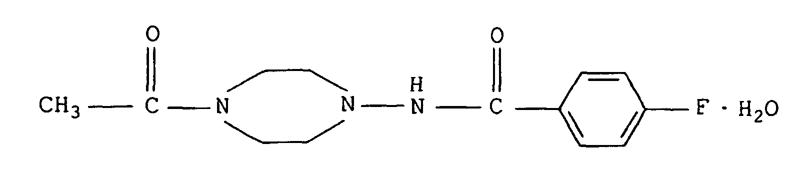 Chemical Name:
N-(4-acetyl-1-piperazinyl)-p-fluorobenzamide monohydrate (hereinafter referred to as FK960).

The antidementics of which the potency is enhanced by the antidementic and/or antiamnesic potency enhancer of the invention are not specifically defined, but are preferably antidementic drugs and others of which the function and the mechanism differ from those of the antidementic and/or antiamnesic potency of the aminopiperazine or aminopiperidine derivatives of the invention, for example, antidementics having an acetylcholine-stimulating activity.

Such antidementics having an acetylcholine-stimulating activity include those having an acetylcholinesterase-inhibiting activity, those having an acetylcholine receptor-agonizing activity, those having a choline-retake promoting activity, etc. Above all, preferred are those having an acetylcholinesterase-inhibiting activity; and more preferred are E2020, tacrine (THA), TAK-147, SDZ-ENA-713, NIK-247, galatamin [See Masaya Hasegawa et al., Folia Pharmacol. Jpn. (Nippon Yakurigaku Zasshi), 114, 327-336 (1999); Tadashi Takahasi et al., Japanese Journal of Geriatric Psychiatry (Ronen Seishin Igaku Zasshi), 10, 593-605 (1999)]; and even more preferred is E2020.

The dose of the active ingredient, aminopiperazine or aminopiperidine derivative (I) shall be suitably selected, depending on various factors such as the type of the other antidementic that is desired to be enhanced, the body weight, the age and the disease condition of the patient, and the administration route, but in general, the oral dose thereof may be from 0.1 to 200 mg/adult at a time.

The administration route of the antidementic and/or antiamnesic potency enhancer of the invention is not specifically defined. It may be administered as an admixture with any other antidementic; or it may be formulated as a preparation separately from any other antidementic and may be administered both at a time or successively in order. The ratio by weight of the other antidementic to the antidementic and/or antiamnesic potency enhancer of the invention varies, depending on the type of that antidementic, but is preferably in a range between 1:100 and 100:1, more preferably between 1:10 and 10:1.

The antidementic and/or antiamnesic potency enhancer of the invention may be orally or parenterally administered to humans, in any form of ordinary pharmaceutical preparations such as capsules, microcapsules, tablets, sugar-coated tablets, granules, powders, troches, pills, suppositories, solutions, suspensions, emulsions, syrups, injections, etc.

The antidementic and/or antiamnesic potency enhancer of the invention may be formulated in any ordinary manner, using various ordinary organic or inorganic carriers for pharmaceutical preparations, for example, vehicles such as sucrose, starch, mannitol, sorbitol, lactose, glucose, cellulose, talc, calcium phosphate, calcium carbonate, etc.; binders such as cellulose, methyl cellulose, hydroxyethyl cellulose, low-substitution hydroxypropyl cellulose, polypropylpyrrolidone, gelatin, gum arabic, polyethylene glycol, sucrose, starch, etc.; disintegrators such as starch, carboxymethyl cellulose, hydroxypropyl starch, sodium hydrogencarbonate, calcium phosphate, calcium citrate, etc.; lubricants such as magnesium stearate, aerosil, talc, sodium laurylsulfate, etc.; flavors such as citric acid, menthol, glycine, orange powder, etc.; preservatives such as sodium benzoate, sodium bisulfite, methylparaben, propylparaben, etc.; stabilizers such as citric acid, sodium citrate, acetic acid, etc.; suspending agents such as methyl cellulose, polyvinylpyrrolidone, aluminium stearate, etc.; dispersants such as hydroxypropylmethyl cellulose, etc.; diluents such as water, etc.; and substrate waxes such as cacao butter, white petrolatum, polyethylene glycol, etc.

The antidementic and/or antiamnesic potency enhancer of the invention significantly enhances the efficacy of antidementics.

To demonstrate the usefulness of the aminopiperazine or aminopiperidine derivatives for use in the invention, pharmaceutical experiment results of typical compounds are shown below.

### Method of Experiment

A solution prepared by dissolving ibotenic acid in physiological saline to have a concentration of 8 µg/µl was injected into the Meynert's basal nuclei of Fischer male rats (n = 12 to 13, 11-weeks old when used in the experiment). One µl of the solution was injected thereinto over a period of 6 minutes, and then the rats were left as they were for 5.minutes to thereby make them have bilateral injury. Three weeks after the treatment, they were used in the experiment. Before the experiment, they were handled for 3 days. In the mneme test, the rats were first put into the light room of a passive avoidance learning test device, and the latent time taken before they entered the dark room was measured (acclimation trial). One hour after the acclimation, the rats were again transferred into the light room, and when they entered the dark room, its door was closed. Then, the rats were made to receive electric shock (0.4 mA, 4 seconds) via the grid of the floor of the dark room (acquirement trial). 24 hours after the acquirement trial, the rats were again transferred into the light room, and the reaction latent time taken before the rats entered the dark room was measured up to at most 300 seconds (retention trial). FK960 and E2020 were separately dissolved in physiological saline (1 ml/kg), and the aqueous solution was intraabdominally administered to the rats just after the acquirement trial. The dose of each of the two substances was determined on the basis of the test results previously made for the efficacy of the single administration of each substance to the same models. Concretely, the low dose of FK960 was 0.1 mg/kg; and the high dose thereof, or that is, the best dose thereof was 1 mg/kg. The low dose of E2020 was 0.1 mg/kg; and the high dose thereof, or that is, the best dose thereof was 0.32 mg/kg.

In the experiment of combined administration of the two substances, FK960 and E2020, they were administered one by one.

### Results of Experiment

The results are given in Table 1.

**Table 1**

| Synergistic Effect in Combined Administration | | |
|---|---|---|
| Test Compound | Dose (mg/kg each) | Reaction Latent Time (sec) |
| Sham Operation | 0 | 277.4 ± 22.6** |
| Meynert's Basal Nuclei Damage | 0 | 89.4 ± 32.4 |
| FK960 + E2020 | 0.1 + 0 | 145.2 ± 36.3 |
| | 0 + 0.1 | 121.2 ± 35.1 |
| | 0.1 + 0.1 | 205.5 ± 31.1** |
| FK960 + E2020 | 1 + 0 | 189.8 ± 35.0** |
| | 0 + 0.32 | 209.7 ± 29.3** |
| | 1 + 0.32 | 241.5 ± 30.9** |
| ++ P < 0.01 (relative to the group of rats with Meynert's basal nuclei damage but with no drug administration). | | |

As is obvious from the results, when FK960 (0.1 mg/kg) and E2020 (0.1 mg/kg), which were ineffective in single administration, were combined, then the combined administration significantly prolonged the reaction latent time in this experiment, and the thus-prolonged reaction latent time in the combined administration is nearly the same as the reaction latent time in the single administration of E2020 (0.32 mg/kg).

In addition, it has been found that, when FK960 (1 mg/kg) is combined with E2020 (0.32 mg/kg), then the combined administration further prolongs the reaction latent time than that in the single administration of each substance. Accordingly, FK960 is extremely useful as an antidementic and/or antiamnesic potency enhancer especially for E2020, and, when the two are combined, their doses may be reduced and their side effects may be reduced.

### Example 1:

Tablets each having the following composition were prepared.

| | |
|---|---|
| FK960 (active ingredient) | 100 mg |
| Lactose | 119 mg |
| Low-substitution hydroxypropyl cellulose | 25 mg |
| Polyvinylpyrrolidone | 5 mg |
| Magnesium stearate | 1 mg |

The active ingredient, lactose and low-substitution hydroxypropyl cellulose were well mixed, to which a 20 % polyvinylpyrrolidone solution in ethanol was added to wet it. Next, this was dried at 45°C and passed through a 20-mesh sieve. The resulting granules were mixed with magnesium stearate, and formed into tablets.

## Claims

1. An antidementic and/or antiamnesic potency enhancer, which comprises an aminopiperazine or aminopiperidine derivative of a formula: 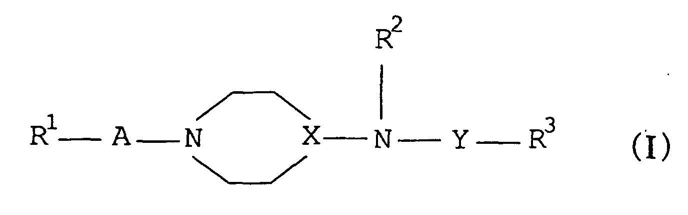 wherein R¹ represents a lower alkyl or an aryl; R² represents hydrogen or a lower alkyl; R³ represents an aryl that may be substituted with a halogen; A represents -CO- or -SO₂-; X represents N or CH; Y represents -CO- or -SO₂-.

2. The antidementic and/or antiamnesic potency enhancer as claimed in claim 1, which enhances the antidementic and/or antiamnesic potency of antidementics having an acetylcholine-stimulating activity.

3. The antidementic and/or antiamnesic potency enhancer as claimed in claim 2, wherein the antidementic having an acetylcholine-stimulating activity is donepedil hydrochloride.
